# EUROPEAN PATENT APPLICATION

(11) **EP 4 198 159 A1**
(43) Date of publication of application: **21.06.2023**
(21) Application number: 21214898.5
(22) Date of filing: 15.12.2021
(51) Int. Cl.: C23C 4/02, A61B 17/32, C23C 4/10, C23C 4/11, C23C 4/129, C23C 4/131, C23C 4/18

(54) **MEDICAL INSTRUMENT AND METHOD FOR MANUFACTURING THE SAME**

(71) Applicant: Aesculap AG, 78532 Tuttlingen (DE)
(72) Inventor: Waeldin, Gilles Roland, 11200 Tanjung Bungah, Pulau Penang (MY); Horn, Christian, 78187 Geisingen (DE); Schweitzer, Tom, 78532 Tuttlingen (DE)
(74) Representative: Patentanwälte Ruff, Wilhelm, Beier, Dauster & Partner mbB

(57) **Abstract**

The invention relates to a method for manufacturing a medical instrument having at least one distal jaw portion with a hardened gripping and/or cutting surface, comprising the steps of: providing the at least one jaw portion; hardening at least one region of the jaw portion by means of: providing a thermal spray device configured for high velocity oxygen fuel spraying; feeding a coating material through the thermal spray device to produce particles of the coating material; impinging the produced particles of coating material at the at least one region of the jaw portion to produce a layer of coating material thereby creating the hardened gripping and/or cutting surface.

## Description

### TECHNICAL FIELD AND PRIOR ART

The invention relates to a medical instrument having at least one distal jaw portion with a hardened gripping and/or cutting surface and to a method for manufacturing such a medical instrument.

Medical instruments having at least one distal jaw portion with a gripping and/or cutting surface are used for gripping, holding, clamping and/or cutting medical material or body tissue and are available in the form of forceps, clamps, surgical needle holders, scissors or the like. To ensure sufficient durability and efficient handling of the medical instrument, the gripping and/or cutting surface of the at least one jaw portion must be sufficiently hard.

For achieving a sufficiently hard or hardened gripping and/or cutting surface it is well-known to attach jaw inserts to the inside of the jaw portion. These jaw inserts are usually pre-shaped and made of tungsten carbide or other hard material. Soldering, welding or other suitable joining techniques are oftentimes used to join the jaw insert to the jaw portion. DE 20 2004 008 169 U1, for example, discloses a method for manufacturing a surgical needle holder with a metal or ceramic jaw insert, wherein the jaw insert is glued to the jaw portion.

The process of joining the jaw insert to the jaw portion by soldering, welding or gluing can involve error-prone manual work. Hence, it may be difficult to achieve sufficient repeatability of the individual steps of the manufacturing process, which can lead to a lack of quality of the medical instrument.

### SUMMARY OF THE INVENTION

It is an object of the invention to provide a method for manufacturing a medical instrument of the type mentioned at the beginning, which allows for improved manufacturing quality and improved properties of the manufactured medical instrument, particularly with respect to the hardened gripping and/or cutting surface.

It is a further object of the invention to provide a medical instrument of the type mentioned at the beginning, which has improved properties, particularly with respect to the hardened gripping and/or cutting surface.

These objects are solved by the method with the features of claim 1 and the medical instrument with the features of claim 10. Preferred embodiments are defined in the dependent claims.

According to a first aspect, a method for manufacturing a medical instrument having at least one distal jaw portion with a hardened gripping and/or cutting surface is provided, the method comprising the steps of: providing the at least one jaw portion; hardening at least one region of the jaw portion by means of: providing a thermal spray device configured for high velocity oxygen fuel spraying (HVOF); feeding a coating material through the thermal spray device to produce particles of the coating material; impinging the produced particles of coating material on the at least one region of the jaw portion to produce a layer of coating material, thereby creating the hardened gripping and/or cutting surface.

For the sake of simplicity, the term "hardened gripping and/or cutting surface" will be abbreviated to "hardened surface" in the following.

The invention eliminates the need of joining a jaw insert to the jaw portion for achieving a hardened surface. Instead, the at least one region of the jaw portion is coated by means of high velocity oxygen fuel spraying (HVOF) to create the hardened surface. The inventors have recognized that creating the hardened surface by means of HVOF allows to achieve superior properties with respect to hardness, durability, surface finishing and accuracy.

High velocity oxygen fuel spraying (HVOF) is a thermal spraying technique. Thermal spraying techniques are coating processes in which partially melted or heated materials are sprayed onto a surface. Thermal spraying can provide relative thick coatings over a large area at high deposition rates as compared to other coating processes such as electroplating, physical and chemical vapor deposition. Coating materials available for thermal spraying include metals, alloys, ceramics, plastics and composite materials. HVOF, in particular, is a special thermal spraying technique in which a mixture of gaseous or liquid fuel and oxygen is fed into a thermal spray device. The mixture of gaseous or liquid fuel and oxygen is ignited and combusted within the thermal spray device. A resultant hot gas stream emanates through a converging-diverging nozzle and travels through a straight section of the thermal spray device. The coating material is fed into the thermal spray device, atomized to particles and accelerated by means of the gas stream. The coating material can be fed into/through the thermal spray device in the form of a powder and/or wire, wherein the resultant particles of coating material are heated and/or partially melted in the stream of hot gas. The stream of hot gas and particles is directed towards the surface to be coated, i.e., the produced particles of coating material are impinged on the at least one region of the jaw portion. Impinging the produced particles of coating material on the at least one region of the jaw portion produces the layer of coating material, i.e., the particles deposit on the surface to be coated. This creates the hardened surface.

The at least one distal jaw portion can be provided as a separate unit or part of the medical instrument. For example, the distal jaw portion can be in the form of a separate jaw member. Alternatively, the distal jaw portion can be provided as an integral portion of a component of the medical instrument, for example of an instrument branch or the like. In one embodiment, the medical instrument has two distal jaw portions forming an instrument mouth. The medical instrument to be manufactured can be in the form of a clamp, forceps, surgical needle holder, scissors or the like for open, endoscopic and/or robotic surgery.

In one embodiment, the layer of coating material is produced with a thickness between 0.1 mm and 2 mm, preferably between 0.2 mm and 1.5 mm, more preferably between 0.4 mm and 0.8 mm. In comparison to other surface coating techniques, such as electroplating, physical and chemical vapor deposition or the like, HVOF allows to deposit a relatively thick layer of coating material. The inventors have recognized that coating the at least one distal jaw portion with a relatively thin layer of coating material can lead to disadvantages. For example, thin coating layers are rather susceptible to breaking during the usage of the medical instrument. This may cause health risks for the patient. Hence, using HVOF for depositing a thick layer of coating material reduces these risks. A thickness between 0.1 mm and 2 mm is advantageous, especially with regard to the required process time. A thickness between 0.2 mm and 1.5 mm is advantageous, especially with regard to the resulting mechanical properties. The inventors have recognized that a thickness between 0.4 mm and 0.8 mm is advantageous, especially with regard to both the required process time for depositing the coating material and for achieving superior mechanical properties of the resulting hardened surface. In one embodiment, the thickness is less than 0.1 mm.

In one embodiment, the method further comprises pre-texturing the at least one region of the jaw portion to provide a non-smooth surface pattern, wherein said pre-texturing is carried out prior to producing the layer of coating material and by means of processing the material of the jaw portion. The non-smooth surface pattern enhances the frictional properties of the hardened surface. Preferably, the non-smooth surface pattern comprises a regular or irregular pattern of recesses, protrusions, teeth or the like. The pre-texturing step is carried out prior to creating the hardened surface. Hence, the at least one region is, at that stage of the method, non-hardened or relatively soft, which allows for a simplified processing of the material of the jaw portion. In one embodiment, the material of the jaw portion is selected from the group consisting of steel, medical grade metal and/or alloys.

In one embodiment, processing the material of the jaw portion involves at least one mechanical machining technique selected from the group consisting of grinding, milling, engraving and embossing and combinations thereof.

In one embodiment, the method further comprises post-texturing the at least one region of the jaw portion to provide a non-smooth surface pattern or to finish the non-smooth surface pattern provided by pre-texturing, wherein said post-texturing is carried out after producing the layer of coating material and by means of processing the layer of coating material. The post-texturing step can be used for both, creating a non-smooth surface pattern for enhanced frictional properties of the hardened surface or for finishing an already existing non-smooth surface pattern created by means of pre-texturing. In contrast to the pre-texturing step, post-texturing is carried out by processing the deposited coating material. Since the coating material is relatively hard, it may be necessary to employ processing techniques different from those used for pre-texturing and subsequent coating by HVOF. Since the post-texturing step is carried out after depositing the coating material, the non-smooth surface pattern can be formed with high accuracy. In comparison to this, creating the non-smooth surface pattern by means of pre-texturing (without an additional post-texturing step) may lead to a loss of accuracy, since the coating material is deposited onto the already existing non-smooth surface pattern. In embodiments not comprising a post-texturing step, preferably the thickness of the layer of coating material is adapted to the shape, form and/or size of the pre-textured non-smooth surface pattern.

In one embodiment, processing the layer of coating material involves at least one electrochemical machining technique selected from the group consisting of pulsed electrochemical machining (PECM), precise electromechanical machining (PEM) and electrochemical milling (ECM) and combinations thereof. Electrochemical machining techniques involve removing material by an electrochemical process and can be used for working extremely hard materials or materials that are difficult to machine using conventional (mechanical) machining techniques. The inventors have recognized that using PECM, PEM and/or ECM for post-texturing is particularly advantageous and allows to create a high accuracy non-smooth surface pattern. In one embodiment, processing the layer of coating material involves at least one mechanical machining technique, preferably milling.

In one embodiment, the coating material is selected from the group consisting of cermets, ceramics, metals, metal alloys, polymers, composites and tungsten carbide. The term "cermet" refers to a composite material composed of ceramic (cer) and metallic (met) materials. A cermet can combine properties of both a ceramic, such as hardness, and those of a metal, such as the ability to undergo plastic deformation. Suitable ceramics include Cr₂O₃, Al₂O₃ and ZrO₂. Suitable (pure) metals include nickel (Ni), copper (Cu), aluminum (Al), molybdenum (Mo) and titanium (Ti). Suitable metal alloys include steel, nickel, chromium and cobalt alloys including NiCrSiB and MCrAIYs. Suitable polymers include polyester and nylon. Suitable composites include nickel graphite. The inventors have found, that the use of tungsten carbide (WC) as coating material offers particular advantages.

In one embodiment, the coating material is a cermet, the cermet comprising at least one metal and at least one ceramic, wherein the at least one metal is selected from the group consisting of cobalt (Co), chromium (Cr), nickel (Ni) and alloys thereof, and wherein the at least one ceramic is selected from the group consisting of tungsten carbide (WC), chromium carbide (Cr₃C₂), silicon boron carbide (SiBC) and mixtures thereof.

In one embodiment, the cermet is a compound comprising or consisting of tungsten carbide (WC) and cobalt (Co) or a compound comprising or consisting of tungsten carbide (WC), cobalt (Co) and chromium (Cr). The inventors have found that using one of the afore-mentioned compounds leads to a hardened surface with superior properties.

According to a second aspect, a medical instrument comprising at least one distal jaw portion is provided, wherein at least one region of the at least one jaw portion comprises a layer of coating material, the layer of coating material forming a hardened surface of the at least one jaw portion. The medical instrument according to the second aspect of the invention is manufactured by means of a method according to the first aspect of the invention. Hence, in order to avoid repetition, reference is made to what has been disclosed with respect to the first aspect. Further features and embodiments of the second aspect follow mutatis mutandis from the disclosure of the first aspect and its embodiments.

### BRIEF DESCRIPTION OF THE DRAWINGS

In the following, embodiments of the invention are described in detail with reference to the drawings. Throughout the drawings, same elements are denoted with same reference signs. The drawings schematically show:
- fig. 1: a perspective view of an embodiment of a medical instrument according to the invention together with an enlarged top view of a hardened gripping and/or cutting surface of the medical instrument;

- fig. 2: a schematic illustration for illustrating an embodiment of a method for manufacturing a medical instrument according to the invention;
- fig. 3: a schematic illustration for illustrating an embodiment of a method according to the invention;
- fig. 4: a schematic illustration for illustrating a further embodiment of a method according to the invention, and
- fig. 5: a schematic illustration for illustrating a further embodiment of a method according to the invention.

### DETAILED DESCRIPTION OF EXEMPLARY EMBODIMENTS OF THE INVENTION

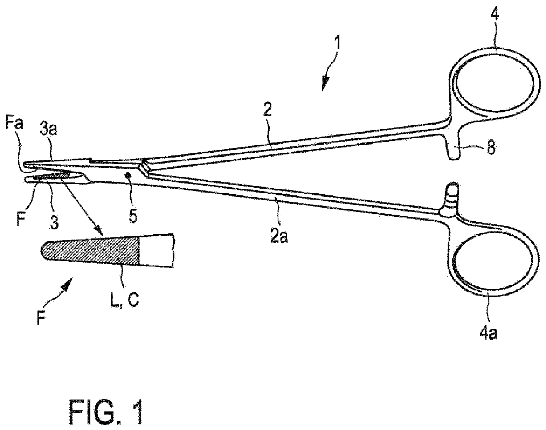

According to fig. 1, a medical instrument 1 comprises two instrument branches 2, 2a, i.e., a first instrument branch 2 and a second instrument branch 2a. Each of the two instrument branches 2, 2a has a distal jaw portion 3, 3a and a proximal handling portion 4, 4a. The jaw portions 3, 3a can also be denoted as first jaw portion 3 and second jaw portion 3a. The handling portions 4, 4a can also be denoted as first handling portion 4 and second handling portion 4a. The two instrument branches 2, 2a are pivotally coupled to each other. In the embodiment shown, the two instrument branches 2, 2a are pivotable relative to each other about a hinge axis 5. The distal jaw portions 3, 3a form an instrument mouth M which is movable between an open state (see fig. 1) and a closed state not depicted in the figures. The instrument mouth M is movable by means of a relative pivotable movement of the two instrument branches 2, 2a about the hinge axis 5. For closing the instrument mouth M, the jaw portions 3, 3a are moved inwardly toward each other. For opening the instrument mouth M, the jaw portions 3, 3a are moved outwardly away from each other.

In the embodiment shown, the medical instrument 1 is a surgical needle holder configured for safely gripping a surgical needle between the distal jaw portions 3, 3a and/or in the instrument mouth M. In other embodiments not shown in the figures, the medical instrument is a clamp, forceps or the like. Put in other words, the medical instrument 1 is forceps-like or clamp-like and allows to grip, grasp, hold and/or clamp medical material or body tissue between the jaw portions 3, 3a.

In an embodiment not shown in the figures, the medical instrument is a surgical scissors configured for cutting medical material or body tissue between the jaw portions. To ensure sufficient durability of the jaw portions 3, 3a and an efficient handling of the medical instrument 1, the jaw portions 3, 3a each comprise a hardened gripping surface F, Fa. Said hardened gripping surfaces F, Fa can also be denoted as first hardened gripping surface F and second hardened gripping surface Fa.

In the afore-mentioned embodiment not shown in the figures, the jaw portions each comprise a hardened cutting surface.

For achieving a sufficiently hard or hardened gripping surface it is well-known in prior art to attach so-called jaw inserts to the inside of the jaw portions. These jaw inserts are usually pre-shaped separate components made of a sufficiently hard material. Soldering, welding or other suitable joining techniques are oftentimes used to join the jaw inserts to the respective jaw portion. This can lead to a rather complicated structure of the medical instrument and to manufacturing methods that require error-prone manual manufacturing steps.

Instead of such jaw inserts, the jaw portions 3, 3a each comprise a layer L of coating material C forming the hardened gripping surface F, Fa of the respective jaw portion 3, 3a. Forming the hardened gripping surfaces F, Fa by means of a coating process which will be described in more detail in the following, allows to simplify the design and manufacturing and to increase the overall quality of the medical instrument 1.

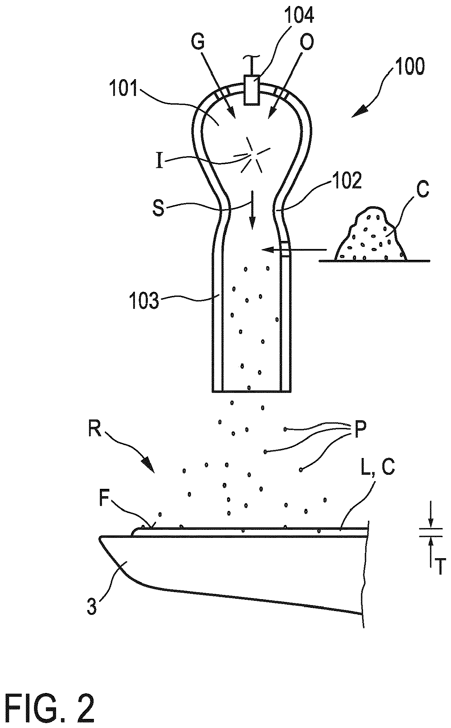

In the following and with reference to figs. 2 to 5, different embodiments of a method for manufacturing the medical instrument 1 are illustrated in more detail. In said figures and the corresponding following description reference is made to the first jaw portion 3 and the first hardened gripping surface F only. However, it goes without saying, that the same manufacturing steps and/or techniques can be used to form the second hardened gripping surface Fa of the second jaw portion 3a. Moreover, the illustrated methods do not only apply to the manufacturing of medical instruments having two jaw portions, but also to medical instruments having only one jaw portion or more than two jaw portions, hence, to medical instruments having at least one jaw portion.

With reference to fig. 2, manufacturing the medical instrument 1 comprises the steps of: providing the (at least one) jaw portion 3 and hardening at least one region R of the jaw portion 3 by means of: providing a thermal spray device 100 configured for high velocity oxygen fuel spraying (HVOF); feeding a coating material C through the thermal spray device 100 to produce particles P of the coating material C; impinging the produced particles P of coating material C on the at least one region R of the jaw portion 3 to produce the layer L of coating material C, thereby creating the hardened gripping surface F.

In one embodiment, the jaw portion 3 is provided together with the (first) instrument branch 2 of the medical instrument. Put in other words, in one embodiment, the distal jaw portion 3 forms the distal end of the instrument branch 2 and is integral with the remaining portions of the instrument branch 2. In other embodiments, the jaw portion 3 is provided as a separate component which is joined to the instrument branch 2 after forming the hardened gripping surface F.

The thermal spray device 100 is configured for HVOF and, in the embodiment shown, comprises a combustion chamber 101, a converging-diverging nozzle 102 and a straight section 103. The converging-diverging nozzle 102 can also be denoted as Laval nozzle and/or supersonic nozzle. The straight section 103 can also be denoted as barrel. The thermal spray device 100 is depicted in a schematic and highly simplified manner.

High velocity oxygen fuel (HVOF) coating is a thermal spray technique which is per se known in the field of surface technology. It involves feeding a gaseous or liquid fuel G and oxygen O into the combustion chamber 101. The mixture of fuel G and oxygen O is ignited by means of an ignition plug 104 and combusted continuously. The ignition and/or combustion I forms a stream S of hot gas that emanates through the converging-diverging nozzle 102 and travels through the straight section 103. In one embodiment, the fuel G is a gaseous fuel and selected from the group consisting of hydrogen, methane, propane, propylene, acetylene, natural gas or mixtures thereof. In another embodiment, the fuel is a liquid fuel, for example kerosene.

In the embodiment shown, the coating material C is fed through the thermal spray device 100 in the form of a powder. More precisely, the coating material C is injected into the stream S of hot gas S right behind the converging-diverging nozzle 102. The stream S of hot gas atomizes the powder and/or the coating material C to particles P. The exit velocity of the particles P exceeds the speed of sound.

The outlet of the straight section 103 is directed towards the region R of the jaw portion. Hence, particles P exiting the outlet impinge the region R and deposit on it by forming said layer L of coating material C, thereby creating the hardened gripping surface F.

In the embodiment shown, the coating material C is a compound of tungsten carbide (WC), cobalt (Co) and chromium (Cr). Said compound is a so-called cermet, i.e., a compound of at least one ceramic (cer) and at least one metal (met). In other embodiments, the coating material is selected from the group consisting of cermets, ceramics, metals, metal alloys, polymers and composite materials.

In a preferred embodiment, the coating material consists of tungsten carbide.

HVOF allows to form coatings considerably thicker than coatings formed by means of common coating techniques, such as electroplating, physical and/or chemical vapor deposition. Hence, the layer L can also be denoted as thick layer. The thickness T of the layer L is between 0.1 mm and 2 mm. In the embodiment shown, the thickness is 0.6 mm.

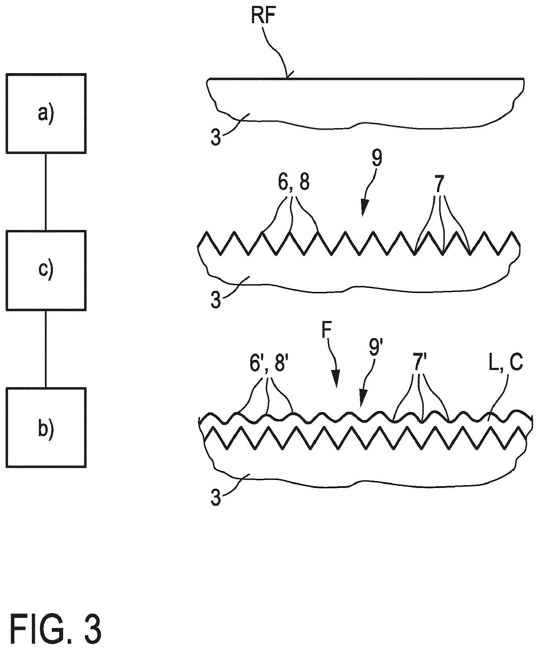

Figs. 3, 4 and 5 schematically illustrate further embodiments.

The method according to fig. 3 comprises the steps of providing the (at least one) jaw portion 3 (step a)) and hardening the region R of the jaw portion 3 (step b)) by means of the HVOF coating technique already illustrated with reference to fig. 2. The method further comprises pre-texturing (step c)) the region R of the jaw portion 3 to provide a non-smooth surface pattern 9, wherein said pre-texturing is carried out prior to creating the hardened gripping surface F and by means of processing the material of the jaw portion 3. The non-smooth surface pattern 9 comprises a regular or irregular pattern of recesses 7 and projections or protrusions 6. Said regular or irregular pattern of recesses 7 and protrusions forms teeth 8. The teeth 8 provide enhanced friction and/or an enlarged surface for depositing the coating material C. The teeth 8 and/or the non-smooth surface pattern 9 are created by means of processing the material of the jaw portion. Prior to pre-texturing, the jaw portion has a smooth surface RF which can also be denoted as raw surface. In the embodiment shown, the material of the jaw portion 3 is a medical grade stainless steel material. In other embodiments, the material of the jaw portion is a medical grade metal alloy or the like.

Processing the material of the jaw portion 3 involves at least one mechanical machining technique selected from the group consisting of grinding, milling, engraving and embossing or combinations thereof.

The hardening by means of HVOF coating (step b)) is carried out after the pre-texturing. Hence, the particles P of the coating material C are impinged onto the non-smooth surface pattern 9 to produce the layer L of coating material C. The layer C covers the non-smooth surface pattern 9, thereby degrading or diminishing the recesses 7 and protrusions 6. Said degradation at least partially preserves the non-smooth surface pattern 9 and forms the hardened gripping surface F with a diminished or degraded non-smooth surface pattern 9′. The diminished or degraded non-smooth surface pattern 9' comprises diminished or degraded recesses 7' and diminished or degraded protrusions 6'. Put in other words, the resulting hardened gripping surface F has teeth 8' slightly rounded off in comparison to the teeth 8.

In the embodiment according to fig. 3, the thickness T of the layer L is adapted to sufficiently preserve the non-smooth surface pattern 9. This allows to arrive at a sufficiently rough and/or non-smooth degraded surface pattern 9' after applying the layer L.

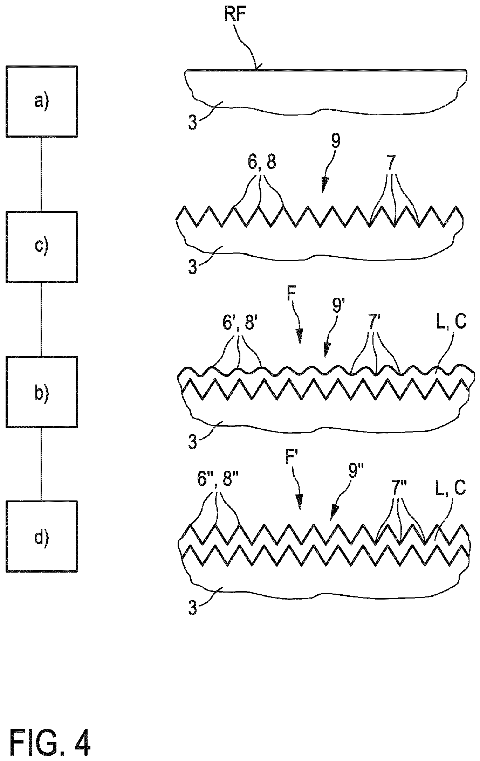

The embodiment illustrated by means of fig. 4 comprises the steps a), c) and b) of the embodiment according to fig. 3. In addition, the embodiment according to fig. 4 comprises a further step of post-texturing (step d)) the region R of the jaw portion 3 to finish and/or enhance the non-smooth surface pattern F provided by pre-texturing and subsequent coating. Said post-texturing is carried out after producing the layer L and by means of processing the layer L of coating material C. Said post-texturing produces a finished hardened gripping surface F'. The finished hardened gripping surface F' can also be denoted as (frictionally) enhanced hardened gripping surface and comprises an enhanced or finished non-smooth surface pattern 9". The enhanced non-smooth surface pattern 9" is produced by processing the layer L created in step b) and forms (in relation to the diminished or degraded protrusions 6' and recesses 7') enhanced protrusions 6" and enhanced recesses 7". Put in other words, the teeth 8 created by machining the material of the jaw portion in step c) are at least partially restored by processing the layer L. In comparison to the non-smooth surface pattern 9', the finished non-smooth surface pattern 9" has enhanced frictional properties and improved accuracy.

Processing the layer L of coating material C in step d) involves at least one electrochemical machining technique selected from the group consisting of pulsed electrochemical machining (PECM), precise electrochemical machining (PEM) and electrochemical milling (ECM) and combinations thereof.

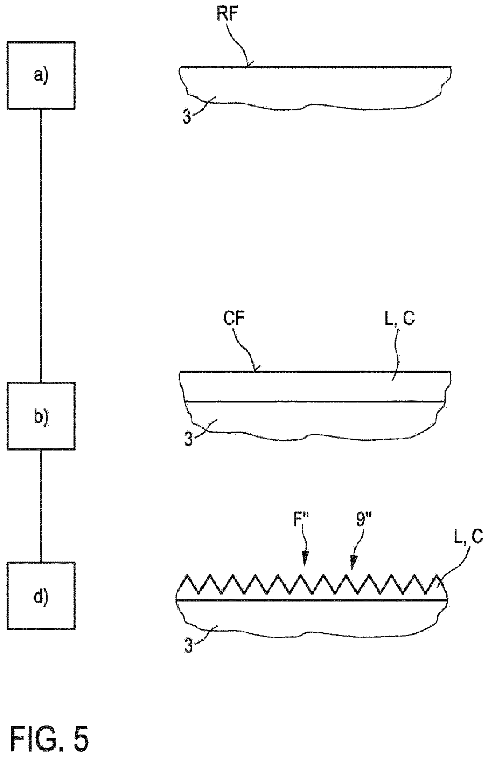

In the embodiment illustrated in fig. 5, the layer L of coating material C is directly applied onto the raw surface RF of the jaw portion forming a smooth coating surface CF. Hence, in contrast to the embodiments of figs. 3 and 4, the embodiment illustrated in fig. 5 does not comprise a pre-texturing of the region R of the jaw portion 3 (step c)). The step of post-texturing (step d)) is carried out to provide a non-smooth surface pattern 9‴ and by means of processing the layer L of coating material. The resulting hardened gripping surface F" is similar to the finished or enhanced hardened gripping surface F' of the embodiment according to fig. 4. In the embodiment according to fig. 5, the layer L is processed by means of at least one electrochemical machining technique.

From the afore-mentioned, a skilled person recognizes that the steps of pre- and post-texturing are advantageous to achieve enhanced frictional properties and improved accuracy of the resultant hardened gripping surface. However, said pre- and/or post-texturing steps are not necessary to carry out the invention.

## Claims

1. A method for manufacturing a medical instrument (1) having at least one distal jaw portion (3) with a hardened gripping and/or cutting surface (F, F', F"), comprising the steps of:
providing the at least one jaw portion (3);
hardening at least one region (R) of the jaw portion (3) by means of:
providing a thermal spray device (100) configured for high velocity oxygen fuel spraying (HVOF);
feeding a coating material (C) through the thermal spray device (100) to produce particles (P) of the coating material (C);
impinging the produced particles (P) of coating material (C) on the at least one region (R) of the jaw portion (3) to produce a layer (L) of coating material (C), thereby creating the hardened gripping and/or cutting surface (F, F', F").

2. The method according to claim 1, **characterized in that** the layer (L) of coating material (C) is produced with a thickness (T) between 0.1 mm and 2 mm, preferably between 0.2 mm and 1.5 mm, more preferably between 0.4 mm and 0.8 mm.

3. The method according to claim 1 or 2, further comprising pre-texturing the at least one region (R) of the jaw portion (3) to provide a non-smooth surface pattern (9), wherein said pre-texturing is carried out prior to producing the layer (L) of coating material (c) and by means of processing the material of the jaw portion (3).

4. The method according to claim 3, **characterized in that** processing the material of the jaw portion (3) involves at least one mechanical machining technique selected from the group consisting of grinding, milling, engraving and embossing and combinations thereof.

5. The method according to any of the preceding claims, further comprising post-texturing the at least one region (R) of the jaw portion to provide a non-smooth surface pattern (9"') or to finish the non-smooth surface pattern (9) provided by pre-texturing, wherein said post-texturing is carried out after producing the layer (L) of coating material (C) and by means of processing the layer (L) of coating material (C).

6. The method according to claim 5, **characterized in that** processing the layer (L) of coating material (C) involves at least one electrochemical machining technique selected from the group consisting of pulsed electrochemical machining (PECM), precise electrochemical machining (PEM) and electrochemical milling (ECM) and combinations thereof.

7. The method according to any of the preceding claims, **characterized in that** the coating material (C) is selected from the group consisting of cermets, ceramics, metals, metal alloys, polymers, composite materials and tungsten carbide.

8. The method according to claim 7, **characterized in that** the coating material (C) is a cermet, the cermet comprising at least one metal and at least one ceramic, wherein the at least one metal is selected from the group consisting of cobalt (Co), chromium (Cr), nickel (Ni) and alloys thereof, and wherein the at least one ceramic is selected from the group consisting of tungsten carbide (WC), chromium carbide (Cr₃C₂), silicon boron carbide (SiBC) and mixtures thereof.

9. The method according to claim 8, **characterized in that** the cermet is a compound comprising or consisting of tungsten carbide (WC) and cobalt (Co) or a compound comprising or consisting of tungsten carbide (WC), cobalt (Co) and chromium (Cr).

10. A medical instrument (1) comprising at least one distal jaw portion (3), **characterized in that** at least one region (R) of the at least one jaw portion (3) comprises a layer (L) of coating material (C), the layer (L) of coating material (C) forming a hardened gripping and/or cutting surface (F, F', F") of the at least one jaw portion (3).
